(19) Europäisches Patentamt

European Patent Office

Office européen des brevets

(11) **EP 1 541 541 B1**

(12) **EUROPÄISCHE PATENTSCHRIFT**

(45) Veröffentlichungstag und Bekanntmachung des Hinweises auf die Patenterteilung:
**27.12.2006 Patentblatt 2006/52**

(51) Int Cl.:
*C07C 29/70* (2006.01)        *C07C 31/30* (2006.01)

(21) Anmeldenummer: **04027035.7**

(22) Anmeldetag: **13.11.2004**

(54) **Verfahren zur Herstellung alkoxyreiner Erdalkalialkoxide**

Process for the preparation of pure alkaline  earth alkoxides

Procédé pour la préparation d' alcoolates alkalino-terreux purs

(84) Benannte Vertragsstaaten:
**AT BE BG CH CY CZ DE DK EE ES FI FR GB GR HU IE IS IT LI LU MC NL PL PT RO SE SI SK TR**

(30) Priorität: **13.12.2003  DE 10358411**

(43) Veröffentlichungstag der Anmeldung:
**15.06.2005  Patentblatt 2005/24**

(73) Patentinhaber: **Degussa AG**
**40474 Düsseldorf (DE)**

(72) Erfinder:
• **Rauleder, Hartwig, Dr.**
  **79618 Rheinfelden (DE)**
• **Standke, Burkhard, Dr.**
  **79540 Lörrach (DE)**

(56) Entgegenhaltungen:
**EP-A- 0 026 547          GB-A- 667 708**
**GB-A- 767 601**

**Beschreibung**

Die Erfindung betrifft ein Verfahren zur Herstellung von Erdalkalidimethanolat, nachfolgend auch Erdalkalimethanolat genannt.

Erdalkalimethanolate werden in vielfältiger Weise in der organisch synthetischen Chemie eingesetzt.

Das gebräuchlichste Verfahren zu ihrer Herstellung ist die direkte Umsetzung von Erdalkalimetall mit Alkohol unter Wasserstoffabspaltung [Liebigs Annalen der Chemie 444, 236 (1925)]. Diese Umsetzung ist bei Verwendung längerkettiger Alkohole aufgrund der geringen Affinität der Reaktionspartner zueinander außerordentlich erschwert.

US 2 965 663 lehrt die Umsetzung von Metallen der Gruppen IA, IIA und IIIA des Periodensystems der Elemente (PSE) mit Alkoholen zu entsprechenden Alkoxiden nach einem speziellen Rückflussverfahren. Nachteilig sind die außerordentlich langen Reaktionszeiten insbesondere bei Einsatz von Metallen der Gruppen IIA und IIIA.

DE-OS 22 61 386 offenbart, daß man die Umsetzung von Erdalkalimetall und Alkohol bei höheren Temperaturen rascher durchführen kann, jedoch mit dem Nachteil, daß man die Umsetzung in einem Autoklaven unter hohem Druck durchführen muss.

Ein generelles Problem bei der Herstellung von Erdalkalialkoxiden aus Erdalkalimetall und Alkohol ist der Restgehalt an nicht umgesetztem Metall, das bei der weiteren Verwendung des Produkts stört, zum Beispiel, wenn man das Magnesiumalkoxid als Katalysator in der organisch synthetischen Chemie einsetzt.

Eine weitere Route zur Herstellung von Metallalkoxiden ist die Alkoholyse (Kirk-Othmer Encyclopedia of Chemical Technology, 3$^{rd}$ Ed., Vol. 2, Seite 8 und 9).

Nach gängiger Lehrmeinung sind in einem Metallalkoxid Alkoxidgruppen durch Alkoxidgruppen eines anderen Alkohols, insbesondere eines höheren Alkohols, zumindest anteilig austauschbar.

Nachteilig bei diesem Verfahren ist, daß bei der Herstellung von Alkoxiden der Gruppe IIA des PSE eine im Produkt verbleibende Verunreinigung an Alkohol beziehungsweise Alkoxid des niedrigeren Alkohols zu beobachten ist. So verbleiben beispielsweise bei der Alkoholyse von Magnesiumethylat, d. h. Magnesiumdiethanolat, mit Isopropanol zu Magnesium-iso-propylat, d. h. Magnesium-di-iso-propanolat, noch ca. 15 Gew.-% Ethanolat gerechnet als Ethanol im Produkt. Auch ein solcher Gehalt an Fremdalkoxid im Produkt kann sich beispielsweise bei der Verwendung von Magnesium-di-iso-propanolat als Katalysator bei einer Synthese in der organischen Chemie als störend auswirken.

Der vorliegenden Erfindung lag somit die Aufgabe zugrunde, ein Verfahren zur Herstellung von metallfreiem Magnesiummethanolat bereitzustellen.

Die Aufgabe wird erfindungsgemäß entsprechend den Merkmalen der Patentansprüche gelöst.

Überraschenderweise wurde gefunden, daß man in einfacher und gleichzeitig wirtschaftlicher Weise ein metallfreies Erdalkalimethanolat der allgemeinen Formel I

$$M(OMe)_2 \qquad (I),$$

worin M für ein Element der zweiten Hauptgruppe des Periodensystems der Elemente steht, vorzugsweise steht M für Magnesium, herstellen kann, wenn man eine Verbindung der allgemeinen Formel II

$$M(OEt)_2 \qquad (II),$$

worin M für ein Element der zweiten Hauptgruppe des Periodensystems der Elemente steht, mit im Überschuss eingesetzten Methanol behandelt, d. h. einer Alkoholyse unterzieht.

Geeigneterweise wird das Reaktionsgemisch zur Durchführung der vorliegenden Umsetzung erwärmt. Vorzugsweise führt man die erfindungsgemäße Umsetzung bei einer Temperatur im Bereich von 20 bis 78 °C unter Normaldruck durch. Die besagte Umsetzung wird in der Regel durch die destillative Entfernung der vorliegenden Alkohole vervollständigt, hierzu kann man auch Vakuum anlegen:

$$M(OEt^2)_2 + x \ MeOH \ \rightarrow \ M(OMe^1)_2 + (x-2) \ HOMe + 2 \ EtOH$$

Man sollte bei der Durchführung des vorliegenden Verfahrens ferner darauf achten, daß das verwendete Destillationssystem über eine hinreichende Trennleistung verfügt.

Unter metallfreien Erdalkalialkoxiden versteht man bei der vorliegenden Erfindung solche, die weniger als 0,04 Gew.-% Erdalkalimetall bezogen auf das Erdalkalialkoxid enthalten. Bei der vorliegenden Erfindung erhält man bevorzugt metallfreie Erdalkalialkoxide der allgemeinen Formel I mit ≤ 0,03 Gew.-% Erdalkalimetall, insbesondere solche mit ≤ 0,02 Gew.-% Erdalkalimetall bis hin zur Nachweisgrenze des betreffenden Erdalkalimetalls, wobei die Angabe jeweils auf das Erdalkalialkoxid bezogen ist.

Gegenstand der vorliegenden Erfindung ist daher ein Verfahren zur Herstellung von einem metallfreien Erdalkalimethanolat der allgemeinen Formel I

$$M(OMe)_2 \qquad (I),$$

worin M für ein Element der zweiten Hauptgruppe des Periodensystems der Elemente steht, durch Alkoholyse einer Verbindung der allgemeinen Formel II

$$M(OEt)_2 \qquad (II),$$

worin M für ein Element der zweiten Hauptgruppe des Periodensystems der Elemente steht, mit im Überschuss eingesetzten Methanol.

Beim erfindungsgemäßen Verfahren entfernt man vorzugsweise während der Umsetzung entstehendes Ethanol oder ein Gemisch aus Methanol und Ethanol destillativ aus dem Reaktionsgemisch.

Im Allgemeinen führt man das erfindungsgemäße Verfahren wie folgt aus:

Zunächst stellt man ein Erdalkaliethanolat, das heißt Erdalkalidiethanolat, in an sich bekannter Weise durch Lösen von Erdalkalimetall in Ethanol - gegebenenfalls unter Erwärmen sowie gegebenenfalls in Gegenwart eines Katalysators - her. Trennt man nach der Umsetzung den überschüssigen Alkohol ab, kann man ein pulverförmiges Metallalkoxid erhalten. Üblicherweise enthält ein so hergestelltes Erdalkalimetallethanolat einen Resterdalkalimetallanteil von ≥ 0,04 Gew.-%, bezogen auf das Erdalkalimetallalkoxid. Üblicherweise handhabt man das Erdalkalialkoxid unter Ausschluss von Feuchtigkeit und unter Schutzgasatmosphäre. Das Erdalkaliethanolat kann als alkoholische Lösung oder in Pulverform für die erfindungsgemäße Alkoholyse eingesetzt werden. Geeigneterweise legt man Methanol, vorzugsweise getrocknetes Methanol, im Überschuss in einem trockenen, kühlbaren beziehungsweise beheizbaren Reaktionsgefäß mit Rührvorrichtung unter Schutzgas, beispielsweise trockener Stickstoff oder Argon, vor und gibt das Edukt - pulverförmig oder dispergiert, teilgelöst oder gelöst in Ethanol und/oder Methanol - zu, wobei man das Reaktionsgemisch vorzugsweise gut mischt und auf eine Temperatur im Bereich von 20 bis 78 °C, vorzugsweise von 40 bis 78 °C, erwärmt. Nach einer Reaktionszeit von 1 bis 8 Stunden kann man den Alkohol bzw. das Alkoholgemisch über die Gasphase, d. h. destillativ aus dem System entfernen. Das gewünschte Produkt fällt in der Regel pulverförmig an und enthält in vorteilhafter Weise weniger als 10 Gew.-% Ethanolat.

Nach dem erfindungsgemäßen Verfahren sind metallfreie Erdalkalimethanolate, d. h. Erdalkalidimethanolat, in einfacher und wirtschaftlicher Weise - auch in einem technischen Maßstab - zugänglich.

Die vorliegende Erfindung wird durch die nachfolgenden Beispiele näher erläutert, ohne den Gegenstand der Erfindung zu beschränken:

## **Beispiele**

Beispiel 1

Herstellung von Magnesiumdimethanolat aus Magnesiumdiethanolat

31 g handelsübliches Magnesiumdiethanolatpulver (Normalkorn, Hersteller Degussa AG) werden mit 500 g Methanol versetzt und auf 40 °C erwärmt. Das Magnesiumethanolat löst sich nahezu vollständig. Anschließend wird bei 68 °C am Rotavapor das alkoholische Lösemittel entfernt. Es bleibt ein weißes Pulver zurück, das nur noch 2 Gew.-% Ethanol (nach Hydrolyse) enthält.

Vergleichsbeispiel A

Herstellung von Magnesium-di-n-propanolat im Autoklaven

In einem 10-l-Stahlautoklaven werden 112 g Magnesiumspäne und 3 000 g Propan-1-ol vorgelegt. Bei 188 °C und einem Druck von 38 bar wird die Umsetzung in insgesamt 5 Stunden durchgeführt. Anschließend wird bei einer Temperatur von 80 °C und einem Druck von ca. 50 mbar n-Propanol destillativ entfernt. Das Alkoxid wird im Anschluss bei 80 °C und einem Druck < 1 mbar getrocknet. Das Produkt enthält noch einen Anteil Mengen von 0,04 Gew.-% metallisches Magnesium.

**Patentansprüche**

1. Verfahren zur Herstellung von einem metallfreien Erdalkalimethanolat der allgemeinen Formel I

$$M(OMe)_2 \qquad (I),$$

worin M für ein Element der zweiten Hauptgruppe des Periodensystems der Elemente steht, durch Alkoholyse einer Verbindung der allgemeinen Formel II

$$M(OEt)_2 \qquad (II),$$

worin M für ein Element der zweiten Hauptgruppe des Periodensystems der Elemente steht, mit im Überschuss eingesetzten Methanol.

2. Verfahren nach Anspruch 1, **dadurch gekennzeichnet, daß** man während der Umsetzung entstehendes Ethanol oder ein Gemisch aus Methanol und Ethanol destillativ aus dem Reaktionsgemisch entfernt.

**Claims**

1. Process for preparing a metal-free alkaline earth methanolate of the general formula I

$$M(OMe)_2 \qquad (I),$$

in which M represents an element from group IIA of the Periodic System of Elements, by alcoholysis of a compound of the general formula II

$$M(OEt)_2 \qquad (II),$$

in which M represents an element from group IIA of the Periodic System of Elements, using excess methanol.

2. Process according to Claim 1, **characterized in that** ethanol or a mixture of methanol and ethanol being produced during reaction is removed from the reaction mixture by distillation.

**Revendications**

1. Procédé de préparation d'un méthanolate alkalino-terreux sans métal de formule générale (I)

$$M(OMe)_2 \qquad (I)$$

dans laquelle M représente un élément du deuxième groupe principal du système périodique des éléments, par alcoolyse d'un composé de formule générale (II)

$$M(OEt)_2 \qquad (II)$$

dans laquelle M représente un élément du deuxième groupe principal du système périodique des éléments, avec du méthanol utilisé en excès.

2. Procédé selon la revendication 1, **caractérisé en ce que** l'éthanol produit durant la réaction ou un mélange de méthanol et d'éthanol est éliminé du mélange réactionnel par distillation.